(19) Europäisches Patentamt
European Patent Office
Office européen des brevets

(11) Publication number: 0 304 318
A2

(12) # EUROPEAN PATENT APPLICATION

(21) Application number: 88307701.8

(22) Date of filing: 19.08.88

(51) Int. Cl.4: C 12 P 7/24
A 61 K 7/46

(30) Priority: 21.08.87 GB 8719832

(43) Date of publication of application:
22.02.89 Bulletin 89/08

(84) Designated Contracting States:
AT BE CH DE ES FR GB GR IT LI LU NL SE

(71) Applicant: NOVALAL LIMITED
8 Rookwood Way
Haverhill Suffolk CB9 8PB (GB)

(72) Inventor: Burfield, Anthony Graham
125 Lion Lane
Haslemere Surrey, GU27 1JI (US)

Best, David John
35 Midland Road
Olney Buckinghamshire, MK46 4BL (GB)

Davis, Karen Johanna
3 High Street South
Olney Buckinghamshire, NK46 4AA (US)

(74) Representative: Stuart, Ian Alexander et al
MEWBURN ELLIS & CO. 2/3 Cursitor Street
London EC4A 1BQ (GB)

Claims for the following Contracting State: ES.
The microorganism(s) has (have) been deposited with National Collection of Industrial and Marine Bacteria under number(s) NCIB 40043.
The applicant has filed a statement in accordance with Rule 28 (4) EPC (issue of a sample only to an expert). Accession number(s) of the deposit(s): NCIB 40043.

(54) **Production of 2-methyl-5-isopropylhexa-2,5-dien-1-al and of 2-methyl-5-isopropyl-hexa-2,4-dien-1-al in microorganisms.**

(57) Microorganisms are employed in the production of 2-methyl-5-isopropyl-hexa-2,5-dien-1-al or 2-methyl-5-isopropyl-hexa-2,4-dien-1-al from α-pinene or its epoxide.

The aldehyde products some of which are novel are of utility in perfume formulations for example in detergent powders and soaps. Microorganisms belonging to the species Pseudomonas fluorescens, and in particular Pseudomonas fluorescens NCIMB 11671 are preferred for effecting the transformations.

EP 0 304 318 A2

## Description

### PRODUCTION OF 2-METHYL-5-ISOPROPYL-HEXA-2,5-DIEN-1-AL AND OF 2-METHYL-5-ISOPROPYL-HEXA-2,4-DIEN-1-AL IN MICROORGANISMS

The present invention relates to the production of 2-methyl-5-isopropyl-hexa-2,5-dien-1-al, and of 2-methyl-5-isopropyl-hexa-2,4-dien-1-al, in microorganisms, for example in Pseudomonas fluorescens NCIMB 11671, its mutants, and related microorganisms.

2-Methyl-5-isopropyl-hexa-2,5-dien-1-al exists in E- and Z-forms as indicated as I and II respectively, these forms being given the trivial names novalal and isonovalal respectively.

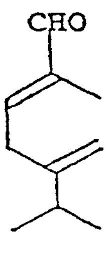
I

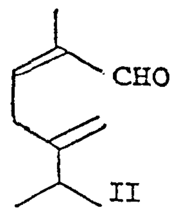
II

Both compounds have distinctive and pleasant smells and are useful as components of perfumes. Novalal has a characteristic lemon-like odour, while isonovalal has a complex odour which has citrus, woody and spicy aspects.

2-methyl-5-isopropyl-hexa-2,4-dien-1-al similarly exists in E- and Z- forms as indicated at III and IV below, these forms being given the trivial names neonovalal and neoisonovalal respectively. These compounds also possess a fragrance which makes them good candidates for perfume formulations.

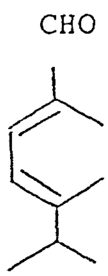
III

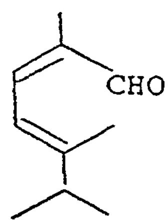
IV

Many microorganisms have been isolated over the past thirty years, which are capable of growth upon the bicyclic terpene hydrocarbon, α-pinene, as sole source of carbon and energy. Pathways for the mineralisation of this compound have largely been inferred by the identification of accumulating metabolites during growth on this hydrocarbon (Shukla,O.P., M.N.Moholay and P.K.Bhattacharrya, Indian Journal of Biochemistry, 5, 79-91 (1968); Shukla,O.P., and P.K.Bhattacharrya, Indian Journal of Biochemistry, 5, 92-101 (1968); Gibbon,G.H. and S.J.Pirt, FEBS Letters, 18, 103-105 (1971); Gibbon,G.H., N.F.Millis and S.J.Pirt, Fermentation Technology Today, 609-612 (1972); Tudroszen,N.J., D.P.Kelly and N.F.Millis, Biochemical Journal, 168, 315-318; Narushima,H., T.Omori and Y.Minoda, European Journal of Applied Microbiology and Bioctechnology, 16, 174-178; Wright,S.J., P.Caunt, D.Carter and P.B.Baker, Applied Microbiology and Biotechnology, 23, 224-227). A variety of metabolites have been described in these studies and include monoterpene hydrocarbons (limonene and terpinolene), oxygenated terpene bodies (cis-thujone, trans-carveol) and various ring-opened acids.

The production of small quantities of L-carvone from α-pinene by fermentation using Pseudomonas sp. NCIMB 11671 is described in EP-A-677682. Low concentrations of L-carvone, of the order of several milligrams per litre, were produced according to the examples of the specification. The organism has been further reclassified by the present applicants as Pseudomonas fluorescens NCIMB 11671. The taxonomy of the microorganism is discussed in EP-A-677682.

The organism has been redeposited in the NCIMB by the present applicants.

The production of novalal from α-pinene by cell-free extracts of Pseudomonas fluorescens NCIMB 11671 has been previously described in a poster presentation at an EMBO workshop 'Genetic Manipulation of

Pseudomonads-Applications in Biotechnology and Medicine', University of Geneva, Geneva, Switzerland, August 31st - September 4th 1986. The work described in the poster was intended to elucidate the metabolic pathway by which L-carvone is produced from α-pinene by fermentation using Pseudomonas fluorescens NCIMB 11671, and established that, in cell free extracts, the first two steps in the degradation pathway of α-pinene involve an epoxidation reaction to produce α-pinene epoxide, followed by a decyclisation reaction, the product of which is 2-methyl-5-isopropyl-hexa-2,5-dien-1-al. The first reaction is catalysed by a pyridine nucleotide dependent monooxygenase, whereas the second rearrangement reaction is independent of any additional cofactor for activity and can function anaerobically, i.e.

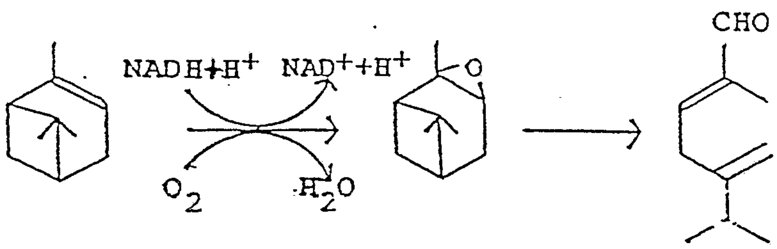


I

The present applicants have now, surprisingly, found that novalal, and its novel isomer isonovalal can be produced in high yields by biotransformation using whole cells. Thus, according to one aspect of the present invention there is provided a method of producing the E and/or Z isomer of 2-methyl-5-isopropyl-hexa-2,5-dien-1-al which comprises contacting a microorganism with α-pinene or, preferably with the epoxide of α-pinene. The substrate for biotransformation can vary from racemic mixtures of optical isomers through to enantiomerically pure materials. Thus (-)-α-pinene, (+)-α-pinene or mixtures of the two may be used. Similarly, epoxides of (-)-α-pinene, (+)-α-pinene or of a mixture of enantiomers may be employed. The Z-isomer (isonovalal) is believed to be the one which is produced naturally by the cells. The ratio of E to Z isomer in the final product appears to depend on the conditions employed in isolating the product, mild conditions such as low temperature, low pressure distillation of the reaction broth favouring the Z-isomer. When the epoxide is used as starting material, it is desirable to carry out the conversion under non-oxidative conditions since no oxygen is required for the conversion and degradative auto-oxidation of the epoxide can thus be avoided.

The neo-isomers of novalal and isonovalal - i.e. 2-methyl-5-isopropyl-hexa-2,4 dien-1-al having E or Z-configuration at the 2- double bond may be produced as further products of this method.

The preferred microorganisms for catalysing the conversions belong to the species Pseudomonas fluorescens. Pseudomonas fluorescens NCIMB 11671 is particularly preferred as are mutants thereof. Other α-pinene metabolising organisms which possess the same degradative pathway would also be suitable. Where the starting material is α-pinene rather than α-pinene epoxide it is desirable to use mutants which have blocked metabolic pathways downstream of 2-methyl-5-isopropyl-hexa-2,5-dien-1-al.

Preferably, the microorganims will have been grown on α-pinene before their use to effect the desired conversion or will have been grown on another carbon source and then induced with α-pinene. Where α-pinene epoxide is used as a starting material it may not be necessary to carry out this step and in the case of Pseudomonas fluorescens NCIMB 11671 satisfactory yields may be obtained when the microorganism has been grown on any of a variety of carbon sources, such as glucose, sucrose, acetate, citrate or succinate.

Non-growing microorganisms are preferred for effecting the conversion; these may be freely suspended or immobilised on a suitable support.

Alternatively, an enzyme preparation may be used in the production of E and/or Z forms of 2-methyl-5-isopropyl-hexa-2,5-dien-1-al from α-pinene epoxide. Again, the proportion of each isomer in the final product is determined by the conditions used to isolate the final product. Isonovalal is believed to be the first product of the enzymic reaction. The enzymes may be free or immobilised and in an aqueous or organic environment. Methods for the immobilisation of enzymes and of microorganisms are known to those skilled in the art and are described for example in "Immobilised Cells and Enzymes: A practical Approach" J Woodward (Ed), IRL Press (1985). Purified enzyme can be obtained by performing a conventional series of chromatographic steps on a cell free extract of cells grown on racemic or optically pure α-pinene, for example (-)-α-pinene. E.g., ion exchange is followed by gel-filtration chromatography.

According to other aspects of the invention, there are provided the novel compounds, isonovalal which is the Z form of 2-methyl-5-isopropyl-hexa-2,5-dien-1-al, neonovalal which is 2-methyl-5-isopropyl-hexa-2,4-dien-1-al having an E- double bond at the 2- position, and neoisonovalal which is 2-methyl-5-isopropyl-hexa-2,4-dien-1-al having a Z-double bond at the 2- position.

According to a further aspect of the invention there is provided a perfume formulation which includes one or more of the novel isomers and a vehicle or diluent therefor.

The previously published work utilising cell free extracts of Pseudomonas fluorescens NCIMB 11671 involved carrying out experiments in conditions optimised for the assay of the first two enzymes in the pathway

and one of the methods of monitoring this activity was the detection of the aldehyde, novalal. Further degradation of this aldehyde would not have been expected because it is possible that the enzyme involved in the further metabolism of the aldehyde is an NAD-linked aldehyde dehydrogenase. As NAD was not supplied and the concentration of endogenous NAD in the cell-free extracts would be very low, aldehyde accumulation resulted. In addition, the conditions of assay for the decyclase are, most probably, different from those required for the maximal activity of the aldehyde dehydrogenase. Therefore, in the cell-free system conditions prevail such that one would expect accumulation of the product of the reaction.

In the present case, however, whole, intact cells of the organism in question are used and, the metabolic pathways are intact and, furthermore subject to close regulation. The usual methods required, in these circumstances, to produce large quantities of primary metabolic intermediates necessitate either a severe manipulation of the environment or the generation of mutant organisms, such that the organism is unable to further metabolise the target metabolite. The transient lifetime of most of these is very short, they are rapidly metabolised and never accumulate to high concentration. In the present case the organism does not need to be subjected to strict environmental manipulation nor is it essential to use a mutant. The overproduction of the two isomers of 2-methyl-5-isopropyl-hexa-2,5-dien-1-al is subject to kinetic regulation. The accumulation of the aldehyde will be regulated by two distinct parameters:

1) the rate of the production reaction (decyclisation);
2) the rate of degradation of the aldehyde.

In the case of the production of the aldehydes from α-pinene epoxide rate (1) is far greater than rate (2) during the production period leading to a quantitative conversion.

The present inventors have found that the decyclisation reaction takes place extremely fast, the ratio of the rates of decyclisation:degradation for the wild type microorganism being surprisingly high.

The present invention will now be described by reference to examples in which<u>Pseudomonas</u> <u>fluorescens</u> NCIMB 11671 is employed to catalyse conversion of starting material to Z-2-methyl-5-isopropyl-hexa-2,5-dien-1-al (hereinafter referred to as isonovalal). Where the E-isomer (hereinafter referred to as novalal) is produced this is due to the conditions used to produce and/or isolate the product.

In the examples below (-)-α-pinene or its epoxide were used as substrates. However (+)-α-pinene or its epoxide could equally well have been employed, as could mixtures of enantiomers.

The organism is cultivated on a minimal salts medium (Gibbon,G.H. and S.J.Pirt, FEBS LETTERS, 18, 103-105) using either α-pinene as the sole carbon source or any of a number of carbon substrates, either alone or in combination with α-pinene. Suitable other substrates include glucose, lactose, sucrose, acetate, succinate, citrate or glutamate. The expression of the requisite enzyme for the decyclisation of α-pinene epoxide is constitutive in this organism and growth on these substrates leads to the production of a catalytically active organism for the bioconversion of α-pinene epoxide. However, exposure of the organism to α-pinene during growth serves to induce higher levels of enzyme activity. Growth of the organism upon sugars or organic acids alone does not induce any level of oxygenase for the initial epoxidation reaction and such organisms cannot be used for the conversion of α-pinene.

The biocatalytic procedure required for the conversion of α-pinene epoxide to the aldehyde involves contacting the organism with the substrate, added either directly to the medium, sonicated in an aqueous medium or in solution in an organic water-soluble carrier, such as ethanol or dimethylformamide. The level of epoxide solution is suitably of the order of 0.32% (v/v), although this may vary. The system is then agitated to ensure equal distribution of the epoxide throughout the aqueous medium until the consumption of the epoxide is complete, usually after three hours incubation at 30°C.

Small amounts of products other than the desired aldehydes, such as α-campholenic aldehyde may arise, due to the autooxidation of the epoxide substrate. One advantage of the process of the invention is that it does not require the presence of molecular oxygen for the decyclisation to take place. The system can therefore be manipulated to reduce the extent of autooxidation by working at very low oxygen tensions. In addition the specificity of the conversion is much improved using induced organisms, as the rate of reaction is much faster, removing substrate before auto-oxidation can occur. The primary product of the enzymatic rearrangement of α-pinene epoxide is the Z-form. 'Isonovalal' may undergo rearrangement, probably non-enzymatically catalysed, <u>in situ</u> in the organism or <u>in vitro</u> during purification. Using whole cells, mixtures of the two aldehydes can be produced, and either isomer can be produced as the predominant product depending on the conditions used for product isolation. Neonovalal and neoisonovalal can also be produced as side products of the rearrangement.

The aldehyde product is extracted from the incubation medium by a single step extraction into Freon 11, followed by removal of the Freon by slight warming, or by other procedures known to the art, such as liquid or dense carbon dioxide, water distillation, ether extraction or absorption.

The various isomers can be distinguished from each other by gas liquid chromatography. GLC is suitably carried out using a 15% SE 30 packed column, 2.1 x 4mm internal diameter, using a column oven temperature of 70°C which is increased at 8°C $min^{-1}$ up to 220°C. An injector temperature of 120°C is suitable. With nitrogen carrier gas and a flow rate of 40ml/min Isonovalal has a retention time of 4.2 mins and Novalal has a retention time of 4.7 mins. The neo-isomers of novalal and isonovalal both have retention times of 6.6 mins under these conditions.

The isomers can also be distinguished by proton nmr by measuring the chemical shift of the aldehyde group relative to a tetramethylsilane standard. For Novalal the aldehyde proton gives a strong signal at around δ

9.3ppm whereas for isonovalal the equivalent proton resonates at around 9.9ppm. Nmr of the neo-isomers indicates the presence of a conjugated double bond, giving rise to resonances in the δ 5-6ppm region, as well as the aldehyde signal.

## EXAMPLES

### 1. Small scale conversion of α-pinene epoxide to 'Novalal'

Pseudomonas fluorescens NCIMB 11671, grown in a minimal salts medium containing α-pinene (0.5% v/v) was resuspended in a sodium phosphate buffer (50 mM, pH 7.0) to an optical density (600 nm) of 1.0, corresponding to a dry weight of 0.3 gm.l.$^{-1}$. This cell suspension (500 ml in a 2 l. conical flask) was incubated with α-pinene epoxide (1.75 ml dispersed in 3.25 ml of phosphate buffer by sonication) for three hours at 30°C on an orbital shaker (150 rpm). After this period the flask was cooled on ice and extracted with two aliquots (200 ml) of Freon 11. These extracts were pooled, dried through anhydrous sodium sulphate and the resultant extract analysed by gas-liquid chromatography for 'Novalal' content. The yield at this stage was 33.6% based on the original quantity of α-pinene epoxide added. The bulk of the Freon extract was concentrated by evaporation over a water bath at 40°C to a volume of 20 ml and the remainder of the solvent removed under a stream of nitrogen at 0°C. This procedure yield 0.312 g of a pale yellow oil, of 96% purity, in a final yield of 17.8%.

### 2. Growth substrate versus biocatalytic ability

A single colony of Pseudomonas fluorescens NCIMB 11671 was removed from a nutrient agar plate and transferred to a universal bottle containing sterile nutrient broth (15 ml). This was incubated over-night to allow the growth of the organism and the resultant culture used for inoculation of the test incubations. Conical flasks (250 ml) containing minimal salts medium (50 ml) were sterilised and to these presterilised solution of one of the following test carbon sources added to give a final concentration of 0.5% (w/v):glucose, sucrose, lactose, acetate, citrate, succinate, glutamate. In addition, cells were also grown on a rich nutrient medium, nutrient broth. Cells were cultivated until the culture optical densities were of the order of 1.5-2.0 at a wavelength of 600 nm. Cultures were then harvested by ultracentrifugation (10,000 g, 30 minutes) and the resultant cell pellets resuspended in sodium phosphate buffer to an optical density of 1.0. Aliquots (5 ml) of these suspensions were then incubated in small conical flasks (25 ml) at 30°C in a shaking water bath (200 rpm) following the addition of (-)-α-pinene epoxide (16 µl). These incubations were continued for three hours, after which the incubations were cooled on ice and extracted into an equal volume of Freon 11. The Freon extract was separated from the aqueous layer and dried through anhydrous sodium sulphate, followed by evaporation to a small volume in a warm water bath. These extracts were then analysed qualitatively by gas-liquid chromatography for the presence of 'Isonovalal'. In all cases examined α-pinene epoxide was converted to 'Isonovalal' or its isomer, indicating that enzyme activity is constitutive.

When Pseudomonas fluorescens NCIMB 11671 was grown on (-)-α-pinene as sole source of carbon and energy, the levels of enzymatic decyclase activity were found to be of the order of three times as great as when grown on glucose as sole source of carbon and energy. Enzymatic decyclase activity was measured in cell-free extracts (prepared as described in worked example 5) of the organism using the following assay system:total assay volume, 1 ml; potassium phosphate buffer (50 µmol, pH 6.0; cell-free extract (0.5-1.5 mg protein); (-)-α-pinene epoxide (0.1 µmoles added in ethanol); phenylhydrazine (1 µmole). The reaction was initiated by the addition of (-)-α-pinene epoxide and the assay incubated at 30°C. The rate of reaction was monitored by following the rate of formation of the hydrazone derivative of the aldehydic product, measured at a wavelength of 320 nm. Under these reaction conditions the activity of glucose grown cells was 0.072 absorbance units. (mg. protein)$^{-1}$•min$^{-1}$ and that of (-)-α-pinene grown organisms, 0.267 absorbance units.(mg. protein)$^{-1}$.min$^{-1}$.

### 3. Use of mutants and other organisms for the same bioconversion

Mutants of Pseudomonas fluorescens can be obtained using two techniques which are well known to the art, in order to generate an organism that is capable of catalysing the conversion of racemic or optically pure α-pinene such as (-)-α-pinene to the aldehyde. In both techniques the activity which is responsible for the further metabolism of the aldehydic product is removed. Since the rate limiting step in conversion of α-pinene to aldehyde is the initial epoxidation of the α-pinene to produce α-pinene epoxide removal of the enzyme downstream of the aldehyde would necessarily cause accumulation of the target aldehyde. The disruption of this reaction sequence can either be achieved by chemical mutagenesis techniques, whereby the organism is exposed to a chemical mutagenic agent such as N-methyl-N'-nitro-N-nitrosoguanidine (NTG) or ethylmethanesulphonate (EMS) or to ultraviolet radiation (see Miller, J.H., Experiments in Molecular Genetics, 1972, Cold Spring Harbour). From this 'damaged' population organisms are selected which are able to accumulate

the aldehyde from the α-pinene, by either screening, under appropriate conditions, for such accumulation or by assaying for the absence, or reduced activity, of the key enzymic activity, the aldehyde dehydrogenase responsible for further oxidation. A more elegant method for the generation of such an organism would be to identify the gene coding for this dehydrogenase and remove it from the organism by genetic engineering techniques.

Using a similar protocol to that described above in example 2 mutants of Pseudomonas fluorescens NCIMB 11671, generated by chemical mutagenesis, have also been tested for their ability to produce the aldehyde. Mutants are believed to have enzymatic lesions downstream of the decyclase activity in the degradation pathway. Two mutants, designated M1/15 and M2/32, both produced the aldehyde under test conditions. Mutants M1/15 and M3/32 were generated from the parent organism by N-methyl-N′-nitro-N-nitrosoguanidine (NTG) mutagenesis and identified by their inability to grow solely on α-pinene and the accumulation of some pinene metabolites when grown on a carbohydrate source in the presence of α-pinene. In addition, other organisms isolated on (-)-α-pinene as sole source of carbon and energy were also effective in this conversion, a representative example being isolate MAL4b, recently identified as another strain of Pseudomonas fluorescens. MAL4b was isolated from soil by selective culture on α-pinene as sole source of carbon and energy.

4. Medium scale aldehyde preparation with α-pinene grown organism

Pseudomonas fluorescens NCIMB 11671, previously grown on α-pinene, was resuspended in sodium phosphate buffer (50 mM, pH 7.0) to an optical density (600 nm) of 1.0, corresponding to a dry weight of 0.3 gm.l.$^{-1}$. This suspension (15 l.) was incubated in a fermentation vessel (nominal capacity 20l.) at 30°C and under agitation (900 rpm). A sonicated emulsion of α-pinene epoxide (53 ml in 47 ml of sodium phosphate buffer) was added to this suspension and the incubation continued for three hours. The cell suspension was pumped from the fermenter vessel into a series of beakers (5 l.) and cooled on ice. The cell suspension (5 x 3 l.) was extracted with diethyl ether (5 x 1.25 l.) followed by n-pentane (5 x 1.15 l.). The solvent extracts were combined and dried over anhydrous sodium sulphate. Small amounts of emulsion formed were broken by centrifugation (10,000 g, 10 minutes, 4°C). Total solvent recovery from these procedures was 11 l. (from an original 11.5 l.). The solvent was evaporated to low bulk (1 l.) over a water bath at 45°C and the remaining solvent stripped under a stream of nitrogen at 0°C. This procedure yielded 38 g of a pale yellow oil, which was shown to be 92.8% pure 'Isonovalal', representing a yield of 58.9%.

5. Examples of product formulations using the novel aldehyde.

'Isonovalal'

'Isonovalal' has a complex odour which reveals citrus, woody, spicy, metallic and resinous aspects:
Citrus: penetrating nitrile and grapefruit character.
Woody: reminiscent of the freshness of PTBCHA, the earthy elements of Vertivert Oil and the balsamic sweetness of Cedarwood Maroc.
Spicy: vaguely reminiscent of cumin, aniseed and the intense sweet note of perilla aldehyde.
Metallic: like the cyclene family of chemicals.
Resinous: becomes more apparent on dry-down and is Oppoponax and Olibanum in character.

The overall fragrance characteristics of the aldehyde may be summarised as aldehydic-woody-fatty.

'Isonovalal' is reasonably powerful, lasting thirty-six hours on smelling strips, and will probably perform equally as a top, middle and end-note material. Its apparent strength suggests a dosage in formulae in the range 1-5% (v/v) and the plurality of its odour indicates use in household perfumery (detergent powders, hard surface cleaners, soaps, etc.). The following formulations have been prepared and assessed; detergent powder fragrance, cologne, hyacinth and apple fragrance (see Tables 1 to 4).

TABLE 1

ISONOVALAL: Perfumery examples.

| | Apple fragrance "A" | Apple fragrance "B" |
|---|---|---|
| OTBCHA[1] ...................... | 18.00 | 18.00 |
| Triplal ..................... | 1.35 | 1.35 |
| Iso-cyclocitral ............ | 1.60 | 1.60 |
| Cis-3-hexenyl acetate ...... | 0.70 | 0.70 |
| Phenyl ethyl iso-butryate .. | 4.50 | 4.50 |
| Citronellol ................ | 11.30 | 11.30 |
| 2-Phenyl ethyl alcohol ..... | 15.70 | 15.70 |
| Hexyl cinnamic aldehyde .... | 22.50 | 22.50 |
| Aldehyde C14 ............... | 4.50 | 4.50 |
| Hedione .................... | 1.10 | 1.10 |
| Lilial ..................... | 1.80 | 1.80 |
| Tonalid .................... | 1.80 | 1.80 |
| Galaxolide 50 .............. | 11.30 | 11.30 |
| Allyl caproate ............. | 1.60 | 1.60 |
| Alpha-damascone 1% in DEP .. | 1.80 | 1.80 |
| Isonovalal ................. | 0.45 | 0.00 |
| Diethyl phthalate .......... | 0.00 | 0.45 |
| Total ...................... | 100.00 | 100.00 |

Notes: Version B replaces Isonovalal with the non-odorous filler Diethyl phthalate.

In this fragrance the Isonovalal has a striking effect, even at such a low percentage. It lifts and freshens the top-note, reducing the sweetness, and creating a natural green apple-peel effect. Overall it lends character, depth and naturalness to the apple fragrance.

1. OTBCHA is ortho-tert-butylcyclohexyl acetate

TABLE 2

ISONOVALAL: Perfumery examples.

| | COLOGNE "A" | COLOGNE "B" |
|---|---|---|
| Lemon Oil .................. | 20.0 | 20.0 |
| Orange Oil ................. | 10.0 | 10.0 |
| Petitgrain oil Paraguay ... | 4.0 | 4.0 |
| Bergamot Synthetic ........ | 15.0 | 15.0 |
| Linalyl Acetate ........... | 15.0 | 15.0 |
| Citral .................... | 10.0 | 10.0 |
| Amyl Cinnamic Aldehyde .... | 7.0 | 7.0 |
| Hedione ................... | 5.0 | 5.0 |
| Eugenol ................... | 2.0 | 2.0 |
| Patchouly Oil ............. | 2.0 | 2.0 |
| Lavandin Oil Super ........ | 5.0 | 5.0 |
| Methyl Ionone ............. | 2.0 | 2.0 |
| Isonovalal ................ | 3.0 | 0.0 |
| Diethyl Phthalate.......... | 0.0 | 3.0 |
| | 100.0 | 100.0 |

Notes: The Isonovalal reduces the overly sweet notes of the lemon and orange in the formula, and introduces a novel woody-grapefruit note.

TABLE 3

ISONOVALAL: Perfumery examples.

| | Detergent powder fragrance "A". | Detergent powder fragrance "B" |
|---|---|---|
| Allyl amyl glycollate 10% in DEP[2] | 0.85 | 0.85 |
| 2-Phenyl ethyl alcohol ......... | 19.50 | 19.50 |
| PTBCHA[3] .......................... | 12.00 | 12.00 |
| Linalol ........................ | 13.50 | 13.50 |
| Cyclacet ....................... | 3.50 | 3.50 |
| Geraniol ....................... | 11.00 | 11.00 |
| Citronellol .................... | 8.00 | 8.00 |
| Petitgrain Paraguay ............ | 2.40 | 2.40 |
| Ylang reconstituted ............ | 0.60 | 0.60 |
| Aldehyde C11-enic 10% in DEP ... | 0.50 | 0.50 |
| Aldehyde C10 10% in DEP ........ | 0.60 | 0.60 |
| Lilial ......................... | 8.00 | 8.00 |
| Galaxolide ..................... | 3.50 | 3.50 |
| Tonalid ........................ | 2.40 | 2.40 |
| Methyl ionone .................. | 1.20 | 1.20 |
| Phenyl ethyl acetate ........... | 1.50 | 1.50 |
| Sandela ........................ | 1.20 | 1.20 |
| Vertofix Coeur ................. | 1.80 | 1.80 |
| Diphenyl oxide ................. | 2.30 | 2.30 |
| Iso e super .................... | 1.80 | 1.80 |
| Eugenol ........................ | 0.85 | 0.85 |
| Fixateur 404 10% in DEP ........ | 0.60 | 0.60 |
| Isonovalal ..................... | 2.40 | 0.00 |
| Diethyl phthalate .............. | 0.00 | 2.40 |
| Total .......................... | 100.00 | 100.00 |

Notes: In version B, Isonovalal is replaced with the non-odorous filler Diethyl phthalate.

In this fragrance the Isonovalal adds freshness and sparkle to the topnote; it enhances the performance of the aldehydes, and lends character and depth to the fragrance.

2. DEP=diethyl phthallate
3. PTBCHA=para-tert-butylcyclohexyl acetate

TABLE 4

ISONOVALAL: Perfumery examples.

| | HYACINTH "A" | HYACINTH "B" |
|---|---|---|
| Benzyl acetate ............. | 10.0 | 10.0 |
| Cinnamic alcohol ........... | 8.0 | 8.0 |
| Benzyl salicylate .......... | 10.0 | 10.0 |
| Eugenol ..................... | 3.0 | 3.0 |
| 2-Phenyl ethyl alcohol ..... | 26.0 | 26.0 |
| Indole ...................... | 4.0 | 4.0 |
| Allyl caproate ............. | 0.5 | 0.5 |
| Galbanum resinoid 50% in DEP | 1.5 | 1.5 |
| Cyclamen aldehyde .......... | 2.0 | 2.0 |
| Exaltolide 10% in DEP....... | 2.0 | 2.0 |
| Phenyl acetaldehyde 50% in PEA[4] | 1.0 | 1.0 |
| Isonovalal ................. | 2.0 | 0.0 |
| Diethyl phthalate .......... | 0.0 | 2.0 |
| Total ...................... | 70.0 | 70.0 |

Notes: Version B replaces Isonovalal with the non-odorous filler Diethyl phthalate.

The Isonovalal rounds off the harsh chemical notes of Hyacinth fragrance, and adds a natural green spiciness to the topnote, and persistence and strength to the end note.

4. PEA is 2-Phenylethylalcohol

**Claims**

1. A method for producing the E and/or Z isomer of 2-methyl-5-isopropyl-hexa-2,5-dien-1-al and/or an isomer of 2-methyl-5-isopropyl-hexa-2,4-dien-1-al having E or Z configuration at the 2- double bond which comprises contacting a microorganism with α-pinene or the epoxide of α-pinene.

2. A method according to claim 1 wherein the microorganism is contacting under non-oxidative conditions with the epoxide of α-pinene.

3. A method according to claim 1 or claim 2 wherein the microorganism belongs to the species Pseudomonas fluorescens.

4. A method according to claim 3 wherein the microorganism is Pseudomonas fluorescens NCIMB 11671 or a mutant thereof.

5. A method according to claim 4 wherein a mutant is used which has metabolic pathways which are blocked downstream of 2-methyl-5-isopropyl-hexa-2,5 dien-1-al.

6. A method according to any one of the preceding claims wherein the microorganism used has been grown on α-pinene or has been grown on another carbon source and then induced with α-pinene prior to its use.

7. A method according to any one of claims 1 to 6 wherein the microorganism is non-growing.

8. A method according to any one of claims 1 to 7 wherein the microorganism is immobilised on a support.

9. A method for producing the E and/or Z isomer of 2-methyl-5-isopropyl-hexa-2,5-dien-1-al and/or an isomer of 2-methyl-5-isopropyl-hexa-2,4-dien-1-al having E or Z configuration at the 2- double bond the method comprising contacting an enzyme preparation from a microorganism grown on α-pinene with α-pinene epoxide.

10. The Z isomer of 2-methyl-5-isopropyl-hexa-2,5-dien-1-al.

11. An isomer of 2-methyl-5-isopropyl-hexa-2,4-dien-1-al having Z configuration at the 2- double bond.

12. An isomer of 2-methyl-5-isopropyl-hexa-2,4-dien-1-al having E configuration at the 2- double bond.

13. A perfume formulation containing a compound of any one of claims 10 to 12 and a vehicle or diluent therefor.

**Claims for the following Contracting State: ES**

1. A method for producing the E and/or Z isomer of 2-methyl-5-isopropyl-hexa-2,5-dien-1-al and/or an isomer of 2-methyl-5-isopropyl-hexa-2,4-dien-1-al having E or Z configuration at the 2- double bond which comprises contacting a microorganism with α-pinene or the epoxide of α-pinene.

2. A method according to claim 1 wherein the microorganism is contacted under non-oxidative conditions with the epoxide of α-pinene.

3. A method according to claim 1 or claim 2 wherein the microorganism belongs to the species Pseudomonas fluorescens.

4. A method according to claim 3 wherein the microorganism is Pseudomonas fluorescens NCIMB 11671 or a mutant thereof.

5. A method according to claim 4 wherein a mutant is used which has metabolic pathways which are blocked downstream of 2-methyl-5-isopropyl-hexa-2,5 dien-1-al.

6. A method according to any one of the preceding claims wherein the microorganism used has been grown on α-pinene or has been grown on another carbon source and then induced with α-pinene prior to its use.

7. A method according to any one of claims 1 to 6 wherein the microorganism is non-growing.

8. A method according to any one of claims 1 to 7 wherein the microorganism is immobilised on a support.

9. A method for producing the E and/or Z isomer of 2-methyl-5-isopropyl-hexa-2,5-dien-1-al and/or an isomer of 2-methyl-5-isopropyl-hexa-2,4-dien-1-al having E or Z configuration at the 2- double bond the method comprising contacting an enzyme preparation from a microorganism grown on α-pinene with α-pinene epoxide.

10. A method comprising production of a compound selected from the Z isomer of 2-methyl-5-isopropyl-hexa-2,5-dien-1-al, an isomer of 2-methyl-5-isopropyl-hexa-2,4-dien-1-al having Z configuration at the 2-double bond, and an isomer of 2-methyl-5-isopropyl-hexa-2,4-dien-1-al having E configuration at the 2-double bond.

11. A method of producing a perfume formulation the method comprising mixing at least one compound of claim 10 with a vehicle or diluent therefor.